# EUROPÄISCHE PATENTSCHRIFT

(11) **EP 1 977 011 B1**
(45) Veröffentlichungstag und Bekanntmachung des Hinweises auf die Patenterteilung: **28.07.2010**
(21) Anmeldenummer: 07703703.4
(22) Anmeldetag: 08.01.2007
(51) Int. Cl.: C12Q 1/68

(54) **VERFAHREN ZUM NACHWEIS VON CYTOSIN-METHYLIERUNGEN**
PROCESS FOR THE DETECTION OF CYTOSINE METHYLATIONS
PROCEDE DE MISE EN EVIDENCE DES METHYLATIONS DE LA CYTOSINE

(30) Priorität: 06.01.2006 DE 102006001161
(43) Veröffentlichungstag der Anmeldung: 08.10.2008
(73) Patentinhaber: Qiagen GmbH, 40724 Hilden (DE)
(72) Erfinder: PEIST, Ralf, 40229 Düsseldorf (DE); TRÄGER, Thorsten, 42781 Haan (DE); FABIS, Roland, 50823 Köln (DE); DEEG, Axel, 42113 Wuppertal (DE)
(86) Internationale Anmeldenummer: PCT/EP2007/050152
(87) Internationale Veröffentlichungsnummer: WO 2007/077262

(56) Entgegenhaltungen:
- WO-A-00/70090
- WO-A-01/98528
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1996, Seiten 5064-5066, XP002106408 ISSN: 0305-1048 in der Anmeldung erwähnt
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048

## Beschreibung

Die vorliegende Erfindung betrifft ein Verfahren zum Nachweis von Cytosin-Methylierungen in DNA.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene, bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die inzwischen am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden; was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Jedoch kann auch dieses Verfahren keine sehr kleinen Fragmente aus geringen Probenmengen zuverlässig analysieren. Diese gehen trotz Diffusionsschutz durch die Matrix verloren.

Die Bisulfit-Technik wird bisher bis auf wenige Ausnahmen (z. B. Zechnigk, M. etal., Eur. J. Hum. Gen. 1997, 5,94-98) nur in der Forschung angewendet. Immer aber werden kurze, spezifische Stücke eines bekannten Gens nach einer Bisulfit-Behandlung amplifziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO-95/00669-A1) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO-99/28498-A2). WO 01/98528 beschreibt ein genomisches Nachweisverfahren.
Für die Abtastung eines immobilisierten DNA-Arrays sind vielfach fluoreszent markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Das Polymerase-Kettenreaktions-Verfahren ("Polymerase Chain Reaction" oder "PCR") ist ein gentechnologisch angewendetes Verfahren, bei dem es gelingt, einige wenige Moleküle einer beliebigen DNA-Sequenz in kurzer Zeit in vitro um Faktoren von 10⁶ bis 10⁸ zu vermehren. Üblicherweise benötigt man dazu zwei synthetisch hergestellte Oligodesoxynukleotid-Primer mit ca. 15 bis 30 Nukleotiden Länge, deren Sequenzen zu den Anfangs- und Endsequenzen der Schwesterstränge der zu vermehrenden ("amplifizierenden") DNA komplementär sind, ein Gemisch aus 4 Desöxynukleotid-5'-triphosphaten, sowie eine thermostabile DNA-Polymerase, die zumindest eine kurzzeitige Erwärmung auf 95°C ohne Funktionseinbuße verträgt.

Für viele Anwendungen ist es nötig, bei einer PCR-Reaktion die Menge an ursprünglich vorhandenen DNA-Molekülen zu bestimmen. Daher gibt es verschiedene Ansätze, die es erlauben, bereits während der einzelnen Schritte der Kettenreaktion die Anzahl der gebildeten DNA-Kopien zu errechnen und daraus auf diesen Wert zu schließen. Meist wird dafür die mittlere, so genannte exponentielle Phase der PCR gewählt, in der sich die Menge an DNA-Template tatsächlich bei jedem Zyklus annähernd verdoppelt.

Matrix-assistierte Laser Desorptions/tonisations-Massenspektrometrie (MALDI-TOF) ist eine sehr leistungsfähige Entwicklung für die Analyse von Biomolekülen (Karas, M. und Hillenkamp, F. (1988), Laserdesorption ionization of proteins with molecular masses exeeding 10000 daltons. Anal. Chem. 60:2299-2301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere. MALDI-TOF Spektroskopie eignet sich ausgezeichnet zur Analyse von Peptiden und Proteinen.

Die Analyse von Nukleinsäuren ist etwas schwieriger (Gut, I. G. und Beck, S. (1 995)), DNA and Matrix Assisted Laser Desorption lonization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter.

In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23:1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

Harnstoff verbessert die Effizienz der Bisulfit- Behandlung vor der Sequenzierung von 5-Methylcytosin in genomischer DNA (Paulin R, Grigg GW, Davey MW, Piper AA.(I 998), Nucleic Acids Res. 26:5009-5010).

Aufgabe der vorliegenden Erfindung ist es, ein verbessertes Verfahren zum Nachweis von Cytosin-Methylierungen in DNA zur Verfügung zu stellen, welches die Nachteile des Standes der Technik überwindet.

Die Aufgabe wird dadurch gelöst, dass ein Verfahren zum Nachweis von Cytosin-Methylierungen in DNA bereit gestellt wird, wobei man folgende Arbeitsschritte ausführt:
a) eine genomische DNA-Probe wird mit einer Lösung eines Bisulfits (=Hydrogensulfit, Disulfit) im Konzentrationsbereich zwischen 0,1 und 6 mol/l inkubiert, wobei Tetrahydrofurfurylalkohol zugegen ist;
b) die erhaltene Reaktionsmischung wird entweder einem Reinigungs- oder Abreicherungsschritt unterworfen, so dass die in Schritt a) zugefügten Reaktanden entfernt werden oder aber die behandelte DNA-Probe wird mit Wasser oder einer wässrigen Lösung verdünnt so dass die in Schritt a) zugefügten Reaktanden die weiteren Reaktionen nicht stören;
e) es wird detektiert, inwieweit sich die Sequenz durch die Behandlung nach Schritt a) gegenüber der genomischen DNA-Probe verändert hat und schließt auf den Methylierungsstatus zumindest eines Locus in der genomischen DNA-Probe.

Die Aufgabe kann auch dadurch gelöst werden, dass ein Verfahren zum Nachweis von Cytosin-Methylierungen in DNA zur Verfügung gestellt wird, wobei man folgende Arbeitsschritte ausführt:
a) eine genomische DNA-Probe wird mit einer Lösung eines Bisulfits (=Hydrogensulfit, Disulfit) im Konzentrationsbereich zwischen 0,1 und 6 mol/l inkubiert, wobei neben mindestens einem Radikalfänger und Tetrahydrofurfurylalkohol, ggf. weitere denaturierende Reagenz(ien) und/oder Lösemittel zugegen sind;
b) die erhaltene Reaktionsmischung wird entweder einem Reinigungs- oder Abreicherungsschritt unterworfen, so dass die in Schritt a) zugefügten Reaktanden entfernt werden oder aber die behandelte DNA-Probe wird mit Wasser oder einer wässrigen Lösung verdünnt so dass die in Schritt a) zugefügten Reaktanden die weiteren Reaktionen nicht stören;
c) ggf. wird die DNA-Probe entweder während des Reinigungs- oder Abreicherungsschritts einem Desulfonierungsschritt unterworfen oder die DNA-Probe wird in einer anschließenden Reaktion einem Desulfonierungsschritt unterworfen
d) ggf. wird die DNA-Probe in einer Polymerasereaktion amplifiziert;
e) es wird detektiert, inwieweit sich die Sequenz durch die Behandlung nach Schritt a) gegenüber der genomischen DNA-Probe verändert hat und schließt auf den Methylierungsstatus zumindest eines Locus in der genomischen DNA-Probe.

Überaschenderweise wurde festgestellt, dass Tetrahydrofurfurylalkohol gegenüber anderen Detergenzien bzw. Lösungsmitteln hinsichtlich Umweltverträglichkeit und Toxizität für die Verwendung des erfindungsgemäßen Verfahrens vorteilhaft gegenüber anderen hier eingesetzten Lösungsmitteln vorteilhaft ist.

Bevorzugt ist es dabei, dass das denaturierende Reagenz und/oder Lösungsmittel aus der folgenden Liste von Verbindungen oder Verbindungsklassen ausgewählt ist: Polyethylenglykoldialkylether, Dioxan und substituierte Derivate, Harnstoff oder Derivate, Acetonitril, primäre Alkohole, sekundäre Alkohole, tertiäre Alkohole, Diethylenglykoldialkylether, Triethylenglykoldialkylether, Tetraethylengtykol-dialkylether, Pentaethylenglykoldiakylether, Hexaethylenglykoldialkylether, DMSO, THF.

Bevorzugt ist weiterhin, dass der Radikalfänger aus der folgenden Gruppe von Verbindungen ausgewählt ist:
Di-, Trihydroxybenzole, Grünteeextrakt (green tea extract), pine bark extract (Pycnogenol), Ginkgo Biloba Extrakt (EGb 761), Flavonoid-Mischung verschiedener Frucht- und Gemüseextrakte (GNLD), Bio-Normalizer (Sun-O Corp), DPPH (1,1-Diphenyl-2-picrylhydrazyl), NDGA (Nordihydroguajaretsäure), Trolox (6-Hydroxy-2,5,7,8-tetramethylchroman-2-carbonsäure), 2,6-Di-tert-butylphenol, 4-Methyl-di-tert-butylphenol, 4-Methoxy-di-tert-butylphenol, 2,6-Di-tert-butyl-p-cresol, 3,4-Dihydroxybenzoesäure, Vitamin C, Vitamin E, Vitamin Q, Hydrochinon, Ubichinon, Lignane, Hydroxyterpene, Flavonoide, Curcumin, Tannine, Retinsäureverbindungen, Ge-132 Bisbetacarboxyethyl germanium sesquioxide, Superoxid Dismutase (SOD), Superoxid Katalase, Alpha-Naphthoflavon, Ginkgo biloba Extrakt (EGb 761), Di(2-methyl-5-chlorophenyl)dithionat und Cu(II)-Derivate, Mebendazol, CS (Chloroform soluble) alkaloidal Extrakt, 4-(3,5-Di-tert-butyl-4-hydroxyphenyl)-3-hydroxy-1,2-naphthochinon, 4-(3,5-Di-tert-butyl-4-hydroxyphenyl)-3-methoxy-1,2-naphthochinon, 4-(3,5-Di-tert-butyl-4-hydroxyphenyl)-1,2-naphthochinon, 2-(3,5-Di-tert-butyl-4-hydroxyphenyl)-3-brom-1,4-naphthochinon, 2-(3,5-Di-tert-butyl-4-hydroxyphenyl)-3-chlor-1,4-naphthochinon, 2-(3,5-Di-tert-butyl-4-hydroxyphenyl)-3-methoxy-1,4-naphthochinon, 2-(3,5-Di-tert-butyl-4-hydroxyphenyl)-3-hydroxy-1,4-naphthochinon, 2-(3,5-Di-tert-butyl-4-hydroxyphenyl)-1,4-naphthochinon, 4-(3,5-Di-tert-butyl-4-hydroxyphenyl)-3-hydroxy-5,5,8,8-tetramethyl-5,6,7,8-tetrahydro-1,2-anthrachinon, 4-(3,5-Di-tert-butyl-4-hydroxyphenyl)-3-methoxy-5,5,8,8-tetramethyl-5,6,7,8-tetrahydro-1,2-anthrachinon, 4-(3,5-Di-tert-butyl-4-hydroxyphenyl)-5,5,8,8-tetramethyl-5,6,7,8-tetrahydro-1,2-anthrachinon, 3-Brom-4-(3,5-di-tert-butyl-4-hydroxyphenyl)-5,5,8,8-tetramethyl-5,6,7,8-tetrahydro-1,2-anthrachinon, 2-(3,5-Di-tert-butyl-4-oxocyclohexa-2,5-dienyliden)-indan-1,3-dion, 2-(3,5-Di-tert-butyl-4-oxocyclohexa-2,5-dienyliden)-3,4-epoxy-3-hydroxy-4-methoxy-3,4-dihydro-2H-naphthalin-1-on, 2-(3,5-Di-tert-butyl-4-oxocyclohexa-2,5-dienyliden)-3,4-epoxy-3,4-dimethoxy-3,4-dihydro-2H-naphthalin-1-on, 2-(3,5-Di-tert-butyl-4-hydroxyphenyl)-indan-1-on, 3,3-Bi-[2-(3,5-di-tert-butyl-4-hydroxyphenyl)-inden-1-on)-3-yl, 2-(3,5-Di-tert-butyl-4-hydroxyphenyl)-3-brom-5,5,8,8-tetramethyl-5,6,7,8-tetrahydro-1,4-anthrachinon, 2-(3,5-Di-tert-butyl-4-hydroxyphenyl)-3-chlor-5,5,8,8-tetramethyl-5,6,7,8-tetrahydro-1,4-anthrachinon, 2-(3,5-Di-tert-butyl-4-hydroxyphenyl)-3-methoxy-5,5,8,8-tetramethyl-5,6,7,8-tetrahydro-1,4-anthrachinon, 2-(3,5-Di-tert-butyl-4-hydroxyphenyl)-3-hydroxy-5,5,8,8-tetramethyl-5,6,7,8-tetrahydro-1,4-anthrachinon, 2-(3,5-Di-tert-butyl-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-5,5,8,8-tetramethyl-5,6,7,8-tetrahydro-1,4-anthrachinon, 2-Brom-3-(3,5-dibrom-4-hydroxyphenyl)-5,5,8,8-tetramethyl-5,6,7,8-tetrahydro-1,4-anthrachinon, 2-Brom-3-(3-brom-5-tert-butyl-4-hydroxyphenyl)-3-hydroxy-5,5,8,8-tetramethyl-5,6,7,8-tetrahydro-1,4-anthrachinon, 3-Brom-2-(3,5-di-tert-butyl-4-hydroxyphenyl)-1,4-anthrachinon, 2-(3,5-Di-tert-butyl-4-hydroxyphenyl)-3-methoxy-1,4-anthrachinon, 2-(3,5-Di-tert-butyl-4-hydroxyphenyl)-3-hydroxy-1,4-anthrachinon, 5,5,8,8-Tetramethyl-5,6,7,8-tetrahydronaphthalin-1,3-diol, 3-Methoxy-5,5,8,8-tetramethyl-5,6,7,8-tetrahydronaphthalin-1-ol, 4-(3-Chlor-5,5,8,8-tetramethyl-1,4-dioxo-1,4,5,6,7,8-hexahydroanthracen-2-yl)-benzoesäure, Methyl-4-(3-chlor-5,5,8,8-tetramethyl-1,4-dioxo-1,4,5,6,7,8-hexahydroanthracen-2-yl)-benzoat, 4-(3-Hydroxy-1,4-dioxo-1,4-dihydronaphthalin-2-yl)-benzoesäure, Methyl-(3-methoxy-1,4-dioxo-1,4-dihydronaphthalin-2-yl)-benzoesäure, 4-(3-Hydroxy-5,5,8,8-tetramethyl-1,4-dioxo-1,4,5,6,7,8-hexahydroanthracen-2-yl)-benzoesäure, Methyl-4-(3-hydroxy-1,4-dioxo-1,4-dihydronaphthalin-2-yl-azo)-benzoat, 4-(3-Hydroxy-5,5,8,8-tetramethyl-1,4-dioxo-1,4,5,6,7,8-hexahydroanthracen-2-yl-azo)-benzoesäure, 3-(3,5-Di-tert-butyl-4-oxocyclohexa-2,5-dienyliden)-5,5,8,8-tetramethyl-5,6,7,8-tetrahydrocyclopenta[b]-naphthalin-1,2-dion, 3-(3,5-Di-tert-butyl-4-oxocyclohexa-2,5-dienyliden)-5,5,8,8-tetramethyl-5,6,7,8-tetrahydroanthracen-3H-1,2,4-trion, 2-(3,5-Di-tert-butyl-4-hydroxyphenyl)-3-methoxy-5,8-dimethyl-1,4-naphthochinon, 2-(3,5-Di-tert-butyl-4-hydroxyphenyl)-3-methoxy-6,7-dimethyl-1,4-naphthochinon, 2-(3,5-Di-tert-butyl-4-hydroxyphenyl)-3-methoxy-5-methyl-1,4-naphthochinon, 2-(3,5-Di-tert-butyl-4-hydroxyphenyl)-2-methoxy-5-methyl-1,4-naphthochinon, 2-(3,5-Di-tert-butyl-4-hydroxyphenyl)-3-methoxy-6-methyl-1,4-naphthochinon, 3-(3,5-Di-tert-butyl-4-hydroxyphenyl)-2-methoxy-6-methyl-1,4-naphthochinon, 2-(3,5-Di-tert-butyl-4-hydroxyphenyl)-3-methoxy-5,6-dimethyl-1,4-naphthochinon, 3-(3,5-Di-tert-butyl-4-hydroxyphenyl)-2-methoxy-6-methyl-1,4-naphthochinon, 2-(3,5-Di-tert-butyl-4-hydroxyphenyl)-3-methoxy-5,6-dimethyl-1,4-naphthochinon, 3-(3,5-Di-tert-butyl-4-hydroxyphenyl)-2-methoxy-5,6- dimethyl-1,4-naphthochinon, 2-(3,5-Di-tert-butyl-4-hydroxyphenyl)-3-methoxy-5,7- dimethyl-1,4-naphthochinon, 3-(3,5-Di-tert-butyl-4-hydroxyphenyl)-2-methoxy-5,7-dimethlyl-1,4-naphthochinon, 2-(3,5-Di-tert-butyl-4-hydroxyphenyl)-3-ethylthio-5-methyl-1,4-naphthochinon, 2-(3,5-Di-tert-butyl-4-hydroxyphenyl)-3-ethylthio-6- methyl-1,4-naphthochinon, 2-(3,5-Di-tert-butyl-4-hydroxyphenyl)-3-hydroxy-5,8-dimethyl-1,4-naphthochinon, 2-(3,5-Di-tert-butyl-4-hydroxyphenyl)-3-hydroxy-6,7-dimethyl-1,4-naphthochinon, 2-(3,5-Di-tert-butyl-4-hydroxyphenyl)-3-hydroxy-5-methyl-1,4-naphthochinon, 3-(3,5-Di-tert-butyl-4-hydroxyphenyl)-2-hydroxy-5-methyl-1,4-naphthochinon, 2-(3,5-Di-tert-butyl-4-hydroxyphenyl)-3-hydroxy-6-methyl-1,4-naphthochinon, 3-(3,5-Di-tert-butyl-4-hydroxyphenyl)-2-hydroxy-6-methyl-1,4-naphthochinon, 2-(3,5-Di-tert-butyl-4-hydroxyphenyl)-3-hydroxy-5,6-dimethyl-1,4-naphthochinon, 2-(3-Brom-5-tert-butyl-4-hydroxyphenyl)-3-hydroxy-5,6-di methyl-1,4-naphthochinon, 3-(3,5-Di-tert-butyl-4-hydroxyphenyl)-2-hydroxy-5,6-dimethyl-1,4-naphthochinon, 2-(3,5-Di-tert-butyl-4-hydroxyphenyl)-3-hydroxy-5,7-dimethyl-1,4-naphthochinon, 3-(3,5-Di-tert-butyl-4-hydroxyphenyl)-2-hydroxy-5,7-dimethyl-1,4-naphthochinon.

Ganz besonders bevorzugt ist Trolox (6-Hydroxy-2,5,7,8-tetramethylchroman-2-carbonsäure).

Wie in der Literatur beschrieben kann das erfindugsgemäße Verfahren bei 4°C bis 90°C durchgeführt werden. Vorzugsweise wird das Verfahren bei einer Temperatur zwischen 35°C und 70°C durchgeführt.

Geeignete Reinigungs- und Abreicherungsreaktionen sind dem Fachmann bekannt. Beispielsweise werden störende Reagenzien mittels Ultrafiltrationsmembranen, Dialyse oder Absorptioschromatographie abgereichert. Es ist aber auch möglich, die behandelte DNA-Probe mit Wasser oder einer wässrigen Lösung soweit zu verdünnen, dass die Reaktiosstoffe, insbesondere das Bisulfit die weiteren Reaktionsschritte nicht nachteilig beeinflussen. Es kann auch vorteilhaft sein, die behandelte DNA-Probe durch Fällung abzutrennen und nach Waschung in wieder aufgelöster Form gesondert dem weiteren Verfahren zuzuführen.

Weiterhin sind geeignete Desulfonierungsreaktionen, insbesondere solche unter Verwendung von geeigneten Basen, die während oder im Anschluss an die Reinigungs- und Abreicherungsreaktionen eingesetzt werden dem Fachmann bekannt.

Bevorzugt denaturiert man die genomische DNA-Probe vor der Behandlung thermisch.

Es kann vorteilhaft sein, den Schritt d) in zwei Teilschritten wie folgt durchzuführen:
d1) man führt eine PCR Präamplifikation durch mit mindestens einem Primerpaar unterschiedlicher Sequenz, die an eine nach Anspruch 1 vorbehandelte DNA-Probe unspezifisch hybridisieren und daher im PCR Schritt mehr als ein Amplifikat ergeben;
d2) man führt eine PCR Amplifikation des in der Präamplifikation gebildeten Produkts durch mit Primern unterschiedlicher Sequenz, die jeweils zu einem Abschnitt der nach Anspruch 1 vorbehandelten DNA-Probe [(+)-Strang oder (-)-Strang] identisch oder revers komplementär sind und die zu amplifizierende DNA spezifisch hybridisieren.

Es kann weiterhin vorteilhaft sein, dass man die Amplifikation von mehreren DNA-Abschnitten in einem Reaktionsgefäß durchführt.

Bevorzugt ist es, dass man für die Polymerasereaktion eine hitzebeständige DNA-Polymerase verwendet.

Besonders ist es bevorzugt, dass man vor Schritt d) des erfindungsgemäßen Verfahrens eine Desulfonierung der DNA durchführt. Es kann jedoch auch vorteilhaft sein, die Desulfonierung der DNA während der Schritte d) oder e) durchzuführen.

Es ist auch bevorzugt, dass man für die Detektion der vorbehandelten DNA die PCR-Produkte auf einen Oligonukleotid Array hybridisiert und man anschließend die folgenden Teilschritte ausführt:
a) die amplifizierte genomische DNA wird an mindestens ein Oligonukleotid unter Ausbildung einer Duplex hybridisiert, wobei besagte hybridisierte Oligonukleotide mit ihrem 3'-Ende unmittelbar oder im Abstand von bis zu 10 Basen an die Positionen angrenzen, die hinsichtlich ihrer Methylierung in der genomischen DNA-Probe zu untersuchen sind;
(b) das Oligonukleotid mit bekannter Sequenz von n Nukleotiden wird mittels einer Polymerase mindestens um ein Nukleotid verlängert, wobei das Nukleotid eine nachweisbare Markierung trägt und die Verlängerung vom Methylierungsstatus des jeweiligen Cytosins in der genomischen DNA-Probe abhängt.

Es ist bevorzugt, dass man für die Detektion der vorbehandelten DNA die PCR-Produkte auf einen Oligonukleotid Array hybridisiert und man anschließend die folgenden Teilschritte ausgeführt:
(a) man hybridisiert einen Satz von Oligonukleotiden an die amplifizierte genomische DNA unter Ausbildung einer Duplex, wobei dieser Satz von Oligonukleotiden aus zwei verschiedenen Spezies besteht und wobei die hybridisierten Oligonukleotide der ersten Spezies mit ihrem 3'-Ende unmittelbar oder im Abstand von bis zu 10 Basen an die Positionen angrenzen; die hinsichtlich ihrer Methylierung in der genomischen DNA-Probe zu untersuchen sind und wobei das zweite Oligonukleotid der zweiten Spezies an eine zweite Region des Zielmoleküls hybridisiert, so dass das 5'-Ende des Oligonukleotids der zweiten Spezies durch eine Lücke von der Größe eines Einzelnukleotides oder bis zu 10 Nukleotiden vom 3'-Ende des hybridisierten Oligonukleotids der ersten Spezies an der Stelle der besagten ausgewählten Position getrennt ist;
(b) das Oligonukleotid der ersten Spezies mit bekannter Sequenz von n Nukleotiden wird mittels einer Polymerase um höchstens die Anzahl von Nukleotiden verlängert, die zwischen dem 3'-Ende des Oligonukleotids der 1. Spezies und dem 5'-Ende des Oligonukleotids der 2. Spezies liegen, wobei die Verlängerung vom Methylierungsstatus des jeweiligen Cytosins in der genomischen DNA-Probe abhängt;
(c) man inkubiert die Oligonukleotide in Gegenwart einer Ligase, wobei das angrenzende, durch die Polymerasereaktion verlängerte Oligonukleotid der ersten Spezies und das Oligonukleotid der zweiten Spezies verbunden werden und man dadurch ein Ligationsprodukt erhält, sofern im vorangehenden Schritt eine Verlängerung des Oligonukleotids der ersten Spezies derart erfolgte, dass nun das 3'-Ende mit vorhandener 3'-Hydroxyfunktion des verlängerten Oligonukleotids unmittelbar an das 5'-Ende des Oligonukleotids der zweiten Spezies angrenzt.

Dabei ist es besonders bevorzugt, dass die verwendeten Oligonukleotide der ersten Spezies und/oder die verwendeten Oligonukleotide der zweiten Spezies entweder nur die Basen T, A und C oder aber die Basen T, A und G enthalten.

Es ist außerdem bevorzugt, dass man für die Detektion der vorbehandelten DNA die PCR-Produkte auf einen Oligonukleotid Array hybridisiert und man anschließend die folgenden Teilschritte ausführt:
(a) man hybridisiert die amplifizierte genomische DNA an mindestens ein Oligonukleotid mit bekannter Sequenz von n Nukleotiden unter Ausbildung einer Duplex, wobei besagte hybridisierte Oligonukleotide mit ihrem 3'-Ende teilweise oder vollständig an die Positionen hybridisieren, die hinsichtlich ihrer Methylierung in der genomischen DNA-Probe zu untersuchen sind;
(b) das Oligonukleotid, sofern es mit seinem 3'-Terminus zuvor ohne Basenfehlpaarungen an die zu untersuchenden Position hybridisierte, wird mittels einer Polymerase mindestens um ein Nukleotid verlängert, wobei mindestens ein Nukleotid eine nachweisbare Markierung trägt und die Verlängerung vom Methylierungsstatus des jeweiligen Cytosins in der genomischen DNA-Probe abhängt.

Es ist auch bevorzugt, dass man die PCR-Produkte und/oder Verlängerungsprodukte und/oder Ligationsprodukte für die Detektion mit einer nachweisbaren Markierung versieht.

Dabei ist es besonders bevorzugt, dass die Markierungen Fluoreszenimarkierungen und/oder dass die Markierungen Radionuklide sind. Besonders bevorzugt ist es dabei, dass die Markierungen der Nukleotide ablösbare Massenmarkierungen sind, die in einem Massenspektrometer nachweisbar sind.

Insbesondere ist es auch bevorzugt, dass man die PCR-Produkte und/oder Verlängerungsprodukte und/oder Ligationsprodukte insgesamt im Massenspektrometer nachweist und somit durch ihre Masse eindeutig charakterisiert sind. Bevorzugt ist es erfindungsgemäß auch, dass man jeweils ein Fragment der PCR-Produkte und/oder Verlängerungsprodukte und/oder Ligationsprodukte im Massenspektrometer nachweist.

Das erfindungsgemäße Verfahren ist bevorzugt auch dadurch gekennzeichnet, dass man das Fragment des PCR-Produkts und/oder Verlängerungsprodukts und/oder Ligationsprodukts durch Verdau mit einer oder mehrerer Exo- oder Endonukleasen erzeugt.

Es ist weiterhin bevorzugt, dass man zur besseren Detektierbarkeit im Massenspektrometer die erzeugten Fragmente mit einer einzelnen positiven oder negativen Nettoladung versieht.

Ganz besonders bevorzugt ist es, dass man die PCR-Produkte und/oder Verlängerungsprodukte und/oder Ligationsprodukte mittels Matrix assistierter Laser Desorptions/Ionisations Massenspektrometrie (MALDI-TOF) oder mittels Elektrospray Massenspektrometrie (ESI) detektiert und visualisiert.

Das erfindungsgemäße Verfahren eignet sich sowohl für DNA aus extrazellulären Quellen als auch für DNA aus Zellen.

Das erfindungsgemäße Verfahren eignet sich insbesondere für Anwendungen, bei denen man die genomische DNA aus einer komplexen DNA-Quelle erhält, wobei besagte Quelle insbesondere Zelllinien, Samenflüssigkeit, Vaginalflüssigkeit, Lungen -und Bronchial-Lavage, Blut, Sputum, Stuhl, Urin, Gehirn-Rückenmarks-Flüssigkeit, und weitere Körperflüssigkeiten, die freeflow extrazelluläre DNA enthalten können, nicht eingebettetes Gewebe oder eingebettetes Gewebe, beispielsweise eingebettetes oder nicht eingebettetes Gewebe von Augen, Darm, Niere, Hirn, Herz, Prostata, Lunge, Brust, Leber, oder anderer Organe oder Muskulatur, histologische Objektträger und alle möglichen Kombinationen hiervon, umfasst.

Ein weiterer Gegenstand der vorliegenden Erfindung ist die Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; ZNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Gegenstand der Erfindung ist auch die Verwendung eines erfindungsgemäßen Verfahrens zur Unterscheidung von Zelltypen oder Geweben oder zur Untersuchung der Zelldifferenzierung.

Ein weiterer Gegenstand der vorliegenden Erfindung ist schließlich ein Kit, enthaltend wenigstens ein Bisulfit enthaltendes Reagenz, Tetrahydrofurfurylalkohol, (ggf. denaturierende Reagenzien und/oder Lösungsmittel), sowie Radikalfänger und ggf. Primer zur Herstellung der Amplifikate, sowie einer Anleitung zur Durchführung eines Assays nach einem erfindungsgemäßen Verfahren.

Die vorliegende Erfindung stellt ein automatisierbares Verfahren zum Nachweis von Methylcytosin bereit, welches nur Pipettierschritte enthält. Dadurch wird die Effizienz bestehender Verfahren in Bezug auf die Einfachheit der Handhabung, die Qualität, die Kosten und vor allem den Durchsatz verbessert.

## Patentansprüche

1. Verfahren zum Nachweis von Cytosin-Methylierungen in DNA, **dadurch gekennzeichnet, dass** man folgende Arbeitsschritte ausführt:
a) eine genomische DNA-Probe wird mit einer Lösung eines Bisulfits (=Hydrogensulfit, Disulfit) im Konzentrationsbereich zwischen 0,1 und 6 mol/l inkubiert, wobei Tetrahydrofurfurylalkohol zugegen ist;
b) die erhaltene Reaktionsmischung wird entweder einem Reinigungs- oder Abreicherungsschritt unterworfen, so dass die in Schritt a) zugefügten Reaktanden entfernt werden oder aber die behandelte DNA-Probe wird mit Wasser oder einer wässrigen Lösung verdünnt so dass die in Schritt a) zugefügten Reaktanden die weiteren Reaktionen nicht stören;
c) es wird detektiert, inwieweit sich die Sequenz durch die Behandlung nach Schritt a) gegenüber der genomischen DNA-Probe verändert hat und schließt auf den Methylierungsstatus zumindest eines Locus in der genomischen DNA-Probe.

2. Verfahren nach Anspruch 1, wobei in Schritt a) mindestens ein Radikalfänger zugegen ist.

3. Verfahren nach einem der vorhergehenden Ansprüche, wobei die DNA-Probe entweder während des Reinigungs- oder Abreicherungsschritts einem Desulfonierungsschritt unterworfen oder die DNA-Probe in einer anschließenden Reaktion einem Desulfonierungsschritt unterworfen wird.

4. Verfahren nach einem der vorhergehenden Ansprüche, wobei die DNA-Probe vor der Detektion in Schritt c) mit einer Polymerasereaktion amplifiziert.

5. Kit zur Durchführung eines Assays nach Anspruch 1, wobei das Kit mindestens ein Bisulfit enthaltendes Reagenz und Tetrahydrofurfurylalkohol umfasst.

6. Kit nach Anspruch 5, wobei das Kit zusätzlich mindestens einen Radikalfänger umfasst.

## Claims

1. Process for detection of cytosine methylations in DNA, **characterized in that** the following working steps are carried out:
a) incubating a genomic DNA sample with a solution of a bisulfite (=hydrogensulfite, disulfite) in the concentration range between 0.1 and 6 mol/l, wherein tetrahydrofurfuryl alcohol is present;
b) subjecting the reaction mixture obtained either to a purification step or to a concentration reduction step, so that the reactants added in step a) are removed, or alternatively the treated DNA sample is diluted with water or an aqueous solution, so that the reactants added in step a) do not disrupt the further reactions;
c) detecting the extent to which the sequence has changed through the treatment according to step a) compared with the genomic DNA sample and inferring the methylation status of at least one locus in the genomic DNA sample.

2. Process according to Claim 1, wherein at least one free-radical scavenger is present in step a).

3. Process according to either of the preceding claims, wherein the DNA sample is either subjected to a desulfonation step during the purification step or the concentration reduction step, or the DNA sample is subjected to a desulfonation step in a subsequent reaction.

4. Process according to any of the preceding claims, wherein the DNA sample is amplified by a polymerase reaction prior to detection in step c).

5. Kit for carrying out an assay according to Claim 1, wherein the kit comprises at least one bisulfite-containing reagent and tetrahydrofurfuryl alcohol.

6. Kit according to Claim 5, wherein the kit additionally comprises at least one radical scavenger.

## Revendications

1. Procédé de détection de méthylations de la cytosine dans un ADN, **caractérisé en ce qu'**on exécute les étapes suivantes :
a) on incube un échantillon d'ADN génomique avec une solution d'un bisulfite (= hydrogénosulfite, disulfite) dans la plage de concentrations comprise entre 0,1 et 6 mol/l, en présence d'alcool tétrahydrofurfurylique ;
b) on soumet le mélange réactionnel obtenu à une étape de purification ou d'enrichissement, de façon à éliminer les réactifs ajoutés dans l'étape a), ou bien on dilue avec de l'eau ou une solution aqueuse l'échantillon d'ADN traité, de façon que les réactifs ajoutés dans l'étape a) ne perturbent pas les réactions ultérieures ;
c) on détecte dans quelle mesure la séquence a été, sous l'effet du traitement selon l'étape a), modifiée par rapport à l'échantillon d'ADN génomique, et on en conclut l'état de méthylation d'au moins un locus dans l'échantillon d'ADN génomique.

2. Procédé selon la revendication 1, dans lequel, dans l'étape a), au moins un fixateur de radicaux est présent.

3. Procédé selon l'une des revendications précédentes, dans lequel l'échantillon d'ADN est soumis à une étape de désulfonation pendant l'étape de purification ou d'enrichissement, ou bien l'échantillon d'ADN est soumis, dans le cadre d'une réaction subséquente, à une étape de désulfonation.

4. Procédé selon l'une des revendications précédentes, dans lequel l'échantillon d'ADN est amplifié avant la détection de l'étape c) par une réaction par polymérase.

5. Trousse pour mettre en oeuvre un essai selon la revendication 1, la trousse comprenant au moins un réactif contenant un bisulfite, et de l'alcool tétrahydrofurfurylique.

6. Trousse selon la revendication 5, la trousse comprenant en outre au moins un fixateur de radicaux.
